(19) Europäisches Patentamt
European Patent Office
Office européen des brevets

(11) Publication number: 0 601 322 A2

# (12) EUROPEAN PATENT APPLICATION

(21) Application number: 93117408.0

(51) Int. Cl.⁵: A61K 31/70, C07H 19/16

(22) Date of filing: 27.10.93

(30) Priority: 27.10.92 JP 312764/92
27.10.92 JP 312765/92

(43) Date of publication of application:
15.06.94 Bulletin 94/24

(84) Designated Contracting States:
AT BE CH DE DK ES FR GB GR IE IT LI LU MC NL PT SE

(71) Applicant: NIPPON ZOKI PHARMACEUTICAL CO., LTD.
1-2, Hiranomachi Nichome
Chuo-ku, Osaka(JP)

(72) Inventor: Yamada, Toshio, c/o Inst. of Bio-Active Science
Nippon Zoki Pharma. Co.,Ltd.,
442-1, Kinashi
Yashiro-cho, Katoh-gun, Hyogo(JP)

(74) Representative: Hansen, Bernd, Dr. Dipl.-Chem. et al
Hoffmann, Eitle & Partner,
Patentanwälte,
Arabellastrasse 4
D-81925 München (DE)

## (54) Adenosindeaminase inhibitor.

(57) The use of a compound represented by the following general formula (I) or a pharmaceutically acceptable salt thereof

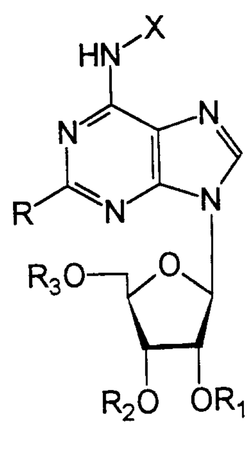

(I)

wherein each of $R_1$, $R_2$ and $R_3$ which may be same or different is hydrogen or alkyl; and R and X have the meanings given in the specification, for the preparation of a medicament which is an adenosinedeaminase inhibitor is disclosed. Some of the compounds of formula (I) are novel compounds. They may be used for the making of a medicament which is for the prevention or therapy of ischemic heart diseases, diseases caused by cerebrovascular disorders, renal diseases and allergic diseases and of post-operative complicated diseases involved with activated oxygen which is generated in ischemic areas during the recirculation of blood after operations.

EP 0 601 322 A2

The present invention relates to adenosinedeaminase inhibitors containing at least one of certain adenosine derivatives and pharmaceutically acceptable salts thereof as an effective component.

Adenosinedeaminase is an enzyme producing inosine by deamination of adenosine in vivo and is widely distributed throughout animals and microorganisms. When said adenosinedeaminase is inhibited, adenosine concentration in tissues is increased while inosine concentration decreases whereupon endogenous inactivation of adenosine is inhibited. When the tissue is in an ischemic state, neutrophils produce activated oxygen and adenosine inhibits said oxygen production and, in addition, it has an action of direct elimination of the produced activated oxygen. Further, as a result of the decrease in the inosine concentration, supply of hypoxanthine which is a substrate in the xanthine-xanthineoxidase system (which is one of the systems producing activated oxygen) is decreased. It has been known that adenosinedeaminase inhibitors which inhibit the production of such activated oxygen sources and also eliminate them exhibit pharmacological actions such as improvement of coronary and cerebral blood vessel circulation, prevention and therapy of renal diseases, anti-inflammatory activity, etc.

The present inventor has conducted investigations on O-alkylated adenosine derivatives, found that the compounds of the present invention exhibit excellent adenosinedeaminase inhibiting action and accomplished the present invention.

One object of the present invention is to provide a novel use of certain adenosine derivatives. A further object of the present invention is to offer a pharmaceutical composition containing at least one of certain adenosine derivatives having adenosinedeaminase inhibiting action as an effective component.

The present invention relates to the use of a compound represented by the following general formula (I) or a pharmaceutically acceptable salt thereof

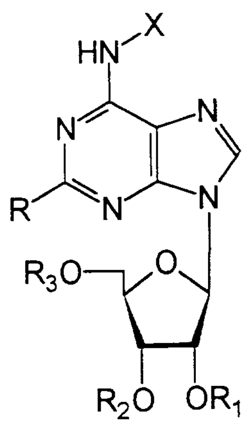

(I)

wherein each of $R_1$, $R_2$ and $R_3$ which may be same or different is hydrogen or alkyl;
R is hydrogen, alkyl, alkenyl, alkynyl, hydroxyalkynyl, alkoxy, phenyl, hydroxy, amino, alkylamino, phenylamino or halogen;
X is hydrogen, alkyl, alkynyl, allyl, methallyl, cycloalkyl, an alkyl group being substituted with one or more hydroxy groups, phenyl (which may be substituted with one or more halogen atoms, lower alkyl groups, lower alkoxy groups and/or trifluoromethyl groups), an alkyl group being substituted with one or more phenyl groups (which may be substituted with one or more halogen atoms, lower alkyl groups, lower alkoxy groups and/or trifluoromethyl groups), bicycloalkyl, naphthylalkyl, acenaphthylenylalkyl or a group represented by the following general formulae (II) or (III)

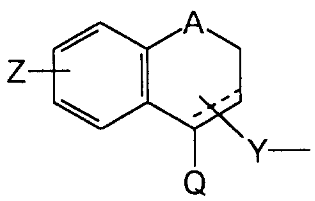

(II)

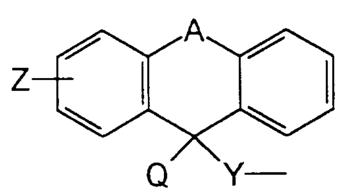

(III)

wherein Z is hydrogen, hydroxy or lower alkoxy;
Q is hydrogen or hydroxy;
A is $-CH_2-$, -O-, -S- or a single bond;
Y is $-(CH_2)_n-$ or a single bond with n being an integer of 1 to 3;
a dashed line means a single bond or a double bond;
and wherein at least one of $R_1$, $R_2$ and $R_3$ is alkyl,
for the preparation of a medicament which is an adenosinedeaminase inhibitor.

In the above general formula (I), each of $R_1$, $R_2$ and $R_3$ may be same or different and is hydrogen or alkyl. Preferably, $R_1$ is hydrogen or a linear or branched alkyl group having 1 to 10 carbon atoms such as methyl, ethyl, propyl, isopropyl, butyl, isobutyl, sec-butyl, tert-butyl, pentyl, isopentyl, neopentyl, tert-pentyl, hexyl, dimethylbutyl, heptyl, octyl, nonyl and decyl, and $R_2$ and $R_3$ are hydrogen or a linear or branched alkyl group having 1 to 4 carbon atoms such as methyl, ethyl, propyl, isopropyl, butyl, isobutyl, sec-butyl and tert-butyl. Most preferably, $R_3$ is hydrogen.

R is hydrogen; alkyl, preferably a linear or branched alkyl group having 1 to 20 carbon atoms such as methyl, ethyl, propyl, isopropyl, butyl, isobutyl, sec-butyl, tert-butyl, pentyl, isopentyl, neopentyl, tert-pentyl, hexyl, dimethylbutyl, heptyl, octyl, nonyl, decyl and stearyl; alkenyl, preferably a linear or branched alkenyl group having 2 to 4 carbon atoms such as ethenyl, 1-propenyl, 2-propenyl, 1-butenyl, 2-butenyl and 3-butenyl; alkynyl, preferably a linear or branched alkynyl group having 2 to 20 carbon atoms such as ethynyl, 1-propynyl, 2-propynyl, 1-butynyl, 2-butynyl, 3-butynyl, sec-butynyl, pentynyl, isopentynyl, 2-pentynyl, 3-pentynyl, 4-pentynyl, hexynyl, heptynyl, octynyl, nonynyl, decynyl and stearynyl; hydroxyalkynyl, preferably one of the above alkynyl groups substituted with one or more hydroxy groups; alkoxy, preferably a linear or branched alkoxy group having 1 to 4 carbon atoms such as methoxy, ethoxy, propoxy, isopropoxy, butoxy, isobutoxy, sec-butoxy and tert-butoxy; phenyl; hydroxy; amino; alkylamino, preferably a linear or branched mono- or dialkylamino group having 1 to 10 carbon atoms such as methylamino, dimethylamino, ethylamino, diethylamino, methylethylamino, propylamino, isopropylamino butylamino, isobutylamino, sec-butylamino, tert-butylamino, pentylamino, isopentylamino, neopentylamino, tert-pentylamino, hexylamino, dimethylbutylamino, heptylamino, octylamino, nonylamino and decylamino; phenylamino; or halogen such as fluorine, chlorine, bromine and iodine. In those symbols, at least one of $R_1$, $R_2$ and $R_3$ is to represent alkyl.

X is hydrogen; alkyl, preferably a linear or branched alkyl group having 1 to 6 carbon atoms such as methyl, ethyl, propyl, isopropyl, butyl, isobutyl, sec-butyl, tert-butyl, pentyl, isopentyl, neopentyl, tert-pentyl, hexyl and dimethylbutyl; alkynyl, preferably a linear or branched alkynyl group having 2 to 7 carbon atoms such as ethynyl, propynyl, butynyl, pentynyl, hexynyl and heptynyl; allyl; methallyl; cycloalkyl, preferably a cycloalkyl group having 3 to 8 carbons such as cyclopropyl, cyclobutyl, cyclopentyl, cyclohexyl, cycloheptyl and cyclooctyl; an alkyl group being substituted with one or more hydroxy groups, preferably a linear or branched alkyl group having 1 to 4 carbon atoms such as methyl, ethyl, propyl, isopropyl, butyl, isobutyl, sec-butyl and tert-butyl substituted with one or two hydroxy groups; phenyl (which may be substituted with one or more halogen atoms, lower alkyl groups, lower alkoxy groups and/or trifluoromethyl groups); an alkyl group being substituted with one or more phenyl groups (which may be substituted with one or more halogen atoms, lower alkyl groups having 1-6 carbon atoms, lower alkoxy groups having 1-6 carbon atoms and/or trifluoromethyl groups), preferably a phenylalkyl or diphenylalkyl group in which one or two optionally substituted phenyl groups as given above is/are bonded to a linear or branched alkyl group having 1 to 3 carbon atoms such as methyl, ethyl, propyl and isopropyl; a bicycloalkyl group, preferably endo- or exo-bicyclo[2,2,1]heptyl; a naphthylalkyl group, wherein preferably an optionally substituted naphthyl group is bonded to an alkyl group having 1 to 3 carbon atoms such as methyl, ethyl, propyl and isopropyl; acenaphthylenylalkyl (including its 1,2-dihydro form), preferably an acenaphthylenylalkyl group in which acenaphthylenyl is bonded to an alkyl group having 1 to 3 carbon atoms such as methyl, ethyl, propyl and isopropyl; or a group represented by the above-mentioned general formula (II) or (III). It is particularly preferable that X is hydrogen or alkyl having 1 to 3 carbon atoms.

In the general formulae (II) and (III), Z is hydrogen, hydroxy or a lower alkoxy group, preferably a linear or branched alkoxy group having 1 to 3 carbon atoms such as methoxy, ethoxy, propoxy and isopropxy; Q is hydrogen or hydroxy; A is $-CH_2-$, -O-, -S- or a single bond; Y is - $(CH_2)_n$- or a single bond; n is an integer of 1 to 3; and a dashed line means a single bond or a double bond.

The said adenosine derivatives of the present invention include novel compounds as follows:

(1) Adenosine derivatives represented by the following general formula (Ia):

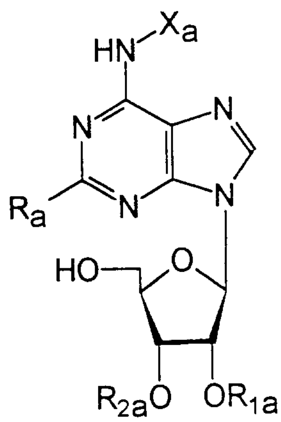

(Ia)

wherein each of $R_{1a}$, $R_{2a}$ and $X_a$ may be same or different and is hydrogen or alkyl; $R_a$ is alkyl having more than 6 carbon atoms, alkenyl, alkynyl, hydroxyalkynyl, alkoxy, phenyl, hydroxy, alkylamino or phenylamino; and at least one of $R_{1a}$ and $R_{2a}$ is alkyl.

(2) Adenosine derivatives represented by the following general formula (Ib)

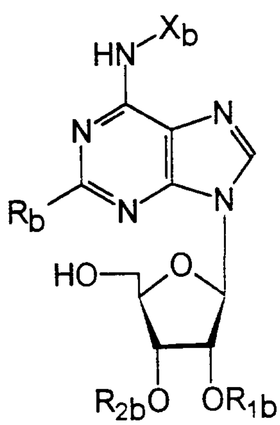

(Ib)

wherein each of $R_{1b}$ and $R_{2b}$ may be same or different and is alkyl; $R_b$ and $X_b$ is hydrogen or alkyl; and at least one of $R_b$ and $X_b$ is alkyl.

(3) Adenosine derivatives represented by the following general formula (Ic)

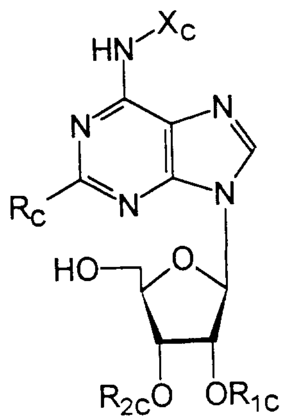

(Ic)

wherein each of $R_{1c}$, $R_{2c}$ and $X_c$ may be same or different and is hydrogen or alkyl ; $R_c$ is bromo or iodo; and at least one of $R_{1c}$ and $R_{2c}$ is alkyl.

(4) Adenosine derivatives represented by the following general formula (Id)

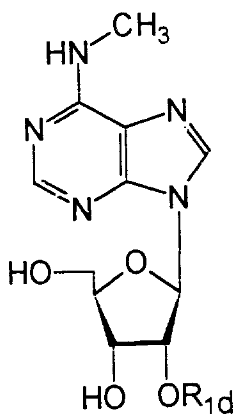

(Id)

wherein $R_{1d}$ is alkyl having more than 2 carbon atoms.

Preferred substituents $R_a$, $R_b$, $R_{1a}$, $R_{1b}$, $R_{2a}$ etc. in the above adenosine derivatives represented by formulae (Ia) to (Id) are the same groups as indicated under formula (I).

Examples of compounds which may be contained as an effective ingredient in the adenosinedeaminase inhibitor in accordance with the present invention are as follows:

(Compound 1) 2'-O-Methyladenosine;
(Compound 2) 3'-O-Methyladenosine;
(Compound 3) 2'-O-Ethyladenosine;
(Compound 4) 2'-O-n-Butyladenosine;
(Compound 5) 2,2'-O-Dimethyladenosine;
(Compound 6) 2,3'-O-Dimethyladenosine;
(Compound 7) 2-Isopropyl-2'-O-methyladenosine;
(Compound 8) 2-Isopropyl-3'-O-methyladenosine
(Compound 9) 2-Methoxy-3'-O-methyladenosine
(Compound 10) 2-Methyl-2'-O-ethyladenosine
(Compound 11) 2-Methyl-2'-O-n-butyladenosine
(Compound 12) 5'-O-Methyladenosine

(Compound 13) 5'-O-n-Butyladenosine
(Compound 14) 2',5'-O-Dimethyladenosine
(Compound 15) 3',5'-O-Dimethyladenosine
(Compound 16) 2,5'-O-Dimethyladenosine
(Compound 17) 2-Methyl-5'-O-n-butyladenosine
(Compound 18) $N^6$,2'-O-Dimethyladenosine
(Compound 19) $N^6$-Ethyl-2'-O-methyladenosine
(Compound 20) $N^6$-n-Butyl-2'-O-methyladenosine
(Compound 21) $N^6$-Methyl-2'-O-ethyladenosine
(Compound 22) $N^6$-Methyl-2'-O-n-butyladenosine
(Compound 23) $N^6$-Methyl-2'-O-n-hexyladenosine
(Compound 24) $N^6$-Methyl-2'-O-n-octyladenosine
(Compound 25) $N^6$,5'-O-Dimethyladenosine
(Compound 26) $N^6$-n-Butyl-5'-O-methyladenosine
(Compound 27) 2,$N^6$,2'-O-Trimethyladenosine
(Compound 28) 2,$N^6$-Dimethyl-2'-O-ethyladenosine
(Compound 29) $N^6$-n-Butyl-2,2'-O-dimethyladenosine
(Compound 30) $N^6$,2'-O-Dimethyl-2-hexyladenosine
(Compound 31) $N^6$,2'-O-Dimethyl-2-decyladenosine
(Compound 32) $N^6$,2'-O-Dimethyl-2-(1-hexyn-1-yl)-adenosine
(Compound 33) $N^6$,2'-O-Dimethyl-2-(1-dodecyn-1-yl)-adenosine
(Compound 34) 2,$N^6$,5'-O-Trimethyladenosine
(Compound 35) $N^6$-n-Butyl-2,5'-O-dimethyladenosine
(Compound 36) 2,$N^6$,3'-O-Trimethyladenosine
(Compound 37) 2-Phenyl-2'-O-methyladenosine
(Compound 38) 2-Phenyl-3'-O-methyladenosine
(Compound 39) 2-Hydroxy-2'-O-methyladenosine
(Compound 40) 2-Hydroxy-3'-O-methyladenosine
(Compound 41) 2-Chloro-2'-O-methyladenosine
(Compound 42) 2-Chloro-3'-O-methyladenosine
(Compound 43) 2-Bromo-2'-O-methyladenosine
(Compound 44) 2-Bromo-3'-O-methyladeonsine
(Compound 45) 2-Bromo-$N^6$,2'-O-dimethyladenosine
(Compound 46) 2-Bromo-$N^6$,3'-O-dimethyladeonsine
(Compound 47) 2-Iodo-2'-O-methyladenosine
(Compound 48) 2-Iodo-3'-O-methyladeonsine
(Compound 49) 2-Fluoro-2'-O-methyladenosine
(Compound 50) 2-Amino-2'-O-methyladenosine
(Compound 51) 2-Amino-3'-O-methyladenosine
(Compound 52) 2-Pentylamino-2'-O-methyladenosine
(Compound 53) 2-Phenylamino-2'-O-methyladenosine
(Compound 54) 2-Phenylamino-3'-O-methyladenosine
(Compound 55) 2-Phenylamino-$N^6$,2'-O-dimethyladenosine
(Compound 56) 2-Phenylamino-$N^6$,3'-O-dimethyladenosine
(Compound 57) 2-(3-Hydroxy-1-propyn-1-yl)-2'-O-methyladenosine
(Compound 58) 2-(3-Hydroxy-1-propyn-1-yl)-3'-O-methyladenosine
(Compound 59) 2',3'-O-Dimethyladenosine
(Compound 60) $N^6$,2',3'-O-Trimethyladenosine
(Compound 61) $N^6$-Methyl-2',3'-O-diethyladenosine
(Compound 62) $N^6$-n-Butyl-2',3'-O-dimethyladenosine
(Compound 63) 2,2',3'-O-Trimethyladenosine
(Compound 64) 2,$N^6$,2',3'-O-Tetramethyladenosine
(Compound 65) $N^6$-Allyl-2'-O-methyladenosine
(Compound 66) $N^6$-Methallyl-2'-O-methyladenosine
(Compound 67) $N^6$-(2,3-Dihydroxypropyl)-2'-O-methyladenosine
(Compound 68) $N^6$-Cyclopropyl-2'-O-methyladenosine
(Compound 69) $N^6$-Cyclopentyl-2'-O-methyladenosine
(Compound 70) $N^6$-Cyclopentyl-2'-O-ethyladenosine

(Compound 71) $N^6$-Cyclopentyl-2,2'-dimethyladenosine
(Compound 72) $N^6$-Cyclopentyl-2-bromo-2'-O-methyl- adenosine
(Compound 73) $N^6$-Cyclohexyl-2'-O-methyladenosine
(Compound 74) $N^6$-Cyclohexyl-2,2'-O-dimethyladenosine
(Compound 75) $N^6$-Cycloheptyl-2'-O-methyladenosine
(Compound 76) $N^6$-p-Methoxyphenyl-2'-O-methyladenosine
(Compound 77) $N^6$-p-Fluorophenyl-2'-O-methyladenosine
(Compound 78) $N^6$-p-Chlorophenyl-2'-O-methyladenosine
(Compound 79) $N^6$-Benzyl-2'-O-methyladenosine
(Compound 80) $N^6$-(R)-Phenyl-isopropyl-2'-O-methyladenoslne
(Compound 81) $N^6$-(2,2-Diphenylethyl)-2'-O-methyl-adenosine
(Compound 82) $N^6$-(exo-Bicyclo[2,2,1]heptyl)-2'-O-methyladenosine
(Compound 83) $N^6$-(endo-Dicyclo[2,2,1]heptyl)-2'-O-methyladenosine
(Compound 84) $N^6$-(1-Naphthyl)methyl-2'-O-methyl-adenosine
(Compound 85) $N^6$-1-(Acenaphthylenyl)methyl-2'-O-methyladenosine
(Compound 86) $N^6$-(1,2-Dihydro-1-acenaphthylenyl)methyl-2'-O-methyladenosine
(Compound 87) $N^6$-(2,3-Dihydro-1H-inden-1-yl)-2'-O-methyladenosine
(Compound 88) $N^6$-(2,3-Dihydro-1H-inden-2-yl)-2'-O-methyladenosine
(Compound 89) $N^6$-(2,3-Dihydro-1H-inden-1-yl)methyl-2'-O-methyladenosine
(Compound 90) $N^6$-(3H-Inden-1-yl)methyl-2'-O-methyladenosine
(Compound 91) $N^6$-(5-Methoxy-2,3-dihydro-1H-inden-2-yl)-2'-O-methyladenosine
(Compound 92) $N^6$-(1-Tetrahydronaphthyl)-2'-O-methyladenosine
(Compound 93) $N^6$-(2-Tetrahydronaphthyl)-2'-O-methyladenosine
(Compound 94) $N^6$-(3,4-Dihydro-1-naphthyl)methyl-2'-O-methyladenosine
(Compound 95) $N^6$-(5-Hydroxy-1-tetrahydronaphthyl)-2'-O-methyladenosine
(Compound 96) $N^6$-(1-Hydroxy-1-tetrahydronaphthyl)- methyl2'-O-methyladenosine
(Compound 97) $N^6$-(5-Methoxyl-1-tetrahydronaphthyl)- 2'-O-methyladenosine
(Compound 98) $N^6$-(6-Methoxy-1-tetrahydronaphthyl)-2'- O-methyladenosine
(Compound 99) $N^6$-(7-Methoxy-1-tetrahydronaphthyl)-2'- O-methyladenosine
(Compound 100) $N^6$-(4-Chromanyl)-2'-O-methyladenosine
(Compound 101) $N^6$-(4-Thiochromanyl)-2'-O-methyl- adenosine
(Compound 102) $N^6$-Fluorenyl-2'-O-methyladenosine
(Compound 103) $N^6$-(9-Fluorenyl)methyl-2'-O-methyladenosine
(Compound 104) $N^6$-(9-Hydroxy-9-fluorenyl)methyl-2'-O-methyladenosine and
(Compound 105) $N^6$-(9-Xanthenyl)methyl-2'-O-methyladenosine.

The compounds of the present invention as given above may be prepared by a method disclosed, for example, in Japanese Laid Open (Kokai) 63/329,294, 02/184,696 and 02/218,689.

The adenosine derivatives of the present invention also include the salts of the compounds represented by the general formulae given above, preferably those being pharmaceutically acceptable. Examples of such salts are acid addition salts such as salts with hydrochloric acid, sulfuric acid, nitric acid, hydrobromic acid, phosphoric acid, perchloric acid, thiocyanic acid, boric acid, formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphthalenesulfonic acid and sulfanilic acid and salts with alkali metals (e.g. sodium and potassium), alkaline earth metals (e.g. calcium and magnesium) and other metals (e.g. aluminum). Adenosine derivatives of the present invention include metal complexes thereof such as, for example, complexes with zinc, nickel, cobalt, copper and iron. Those salts and metal complexes may be manufactured from free adenosine derivatives of the present invention or may be transformed into each other.

When there are stereoisomers for the compounds of the present invention such as cis-trans isomers, optical isomers and conformational isomers, the present invention includes all of them.

The following descriptions serve as illustrative examples for the preparation of compounds of the present invention.

Example 1

10 g of 6-chloro-9-(3,5-O-TIPDS)-$\beta$-D-ribofuranosyl-9H-purine was dissolved in 50 ml of ethyl iodide, and silver oxide was added and stirred with heating. The reaction mixture was applied on a silica gel column, washed with benzene and then eluted with ethyl acetate/hexane. The eluate was concentrated to

dryness under reduced pressure. The residue was dissolved in benzene and a 40%(w/v) aqueous solution of monomethylamine was added thereto. After stirring overnight, the benzene layer was separated, washed with 1N HCl and brine, and a mixture of 1M tetra-n-butylammonium and tetrahydrofuran was added thereto. The reaction mixture was stirred for 3O minutes at room temperature, concentrated under reduced pressure, and then purified by silica gel column chromatography to give 2.1 g of $N^6$-methyl-2'-O-ethyladenosine (Compound 21).

1H-NMR ($D_2O$) : 1.21(3H,t), 3.19(3H,s), 3.58(1H,m), 3.69(1H,m), 3.85(1H,dd), 3.93(1H,dd), 4.32(1H,m), 4.55-(1H,dd), 4.63(1H,dd), 6.10(1H,dd), 8.25(1H,s), 8.3O(1H,s)

In the same manner as mentioned above, the following compounds were obtained.

$N^6$-Methyl-2'-O-n-butyladenosine (Compound 22)

1H-NMR (DMSO-$d_6$):

0.74(3H,t), 1.17(2H,m), 1.37(2H,m), 2.96(3H,s), 3.34(1H,m), 3.55(2H,m), 3.68(1H,m), 3.99(1H,m), 4.29(1H,m), 4.47(1H,m), 5.15(1H,d), 5.42(1H,m), 5.99(1H,d), 7.81(1H,s), 8.23(1H,s), 8.37(1H,s)

$N^6$-Methyl-2'-O-n-hexyladenosine (Compound 23)

1H-NMR ($CDCl_3$):

0.86(3H,t), 1.17(8H,m), 1.40(2H,m), 3.21(3H,s), 3.34(1H,m), 3.48(1H,m), 3.75(1H,m), 3.97(1H,m), 4.36(1H,m), 4.53(1H,d), 4.82(1H,dd), 5.82(1H,d), 5.87(1H,s), 6.87(1H,dd), 7.76(1H,s), 8.37(1H,s)

$N^6$-Methyl-2'-O-n-octyladenosine (Compound 24)

1H-NMR ($CDCl_3$):

0.86(3H,t), 1.16(8H,m), 1.24(2H,m), 1.41(2H,m), 3.21(3H,s), 3.33(1H,m), 3.49(1H,m), 3.76(1H,m), 3.97(1H,m), 4.36(1H,m), 4.53(1H,d), 4.82(1H,dd), 5.82(1H,d), 6.02(1H,s), 6.87(1H,dd), 7.77(1H,s), 8.37(1H,s)

Example 2

5.34 g of adenosine was dissolved in 80 ml of dimethylformamide and 800 mg of 60%(w/w) sodium hydride in mineral oil was added thereto. After stirring for 30 minutes in ice-cold water, 2.84 g of methyl iodide in 10 ml of dimethylformamide was added dropwise. The reaction mixture was stirred under cooling for 2 hours and concentrated to dryness under reduced pressure. The residue was dissolved in water and applied on a cation exchange column. The fraction eluted with a 10%(v/v) aqueous solution of methanol was collected, concentrated to dryness, and purified by silica gel column chromatography to give 1.36 g of 2',3'-O-dimethyladenosine.

m.p.: 180 °C

1H-NMR (DMSO-$d_6$):

3.32(3H,s), 3.40(3H,s), 3.57(1H,m), 3.70(1H,m), 4.09(2H,m), 4.54(1H,dd), 5.47(1H,dd), 6.00(1H,d), 7.35-(2H,s,$D_2O$-Disappearance), 8.14(1H,s), 8.38(1H,s)

Example 3

2 g of the compound obtained in Example 2 and 2 ml of methyl iodide were dissolved in dimethylacetamide, and stirred overnight at room temperature. The reaction mixture was evaporated to dryness under reduced pressure and 10 ml of 2N sodium hydroxide solution was added thereto. The solution was refluxed for one hour with heating. After cooling to room temperature, the solution was reutralized with 2N HCl and applied on an Amberlite XAD-7 column. The column was washed with water and eluted with a 50%(v/v) aqueous solution of methanol. The eluate was concentrated to dryness under reduced pressure and recrystallized from ethyl acetate to give 1.8 g of $N^6$,2',3'-O-trimethyladenosine (Compound 60); m.p.: 165 °C

1H-NMR (DMSO-$d_6$): 2.96(3H,s), 3.31(3H,s), 3.40(3H,s), 3.57(1H,m), 3.70(1H,m), 4.09(2H,m), 4.54(1H,dd), 5.49(1H,dd), 6.01(1H,d), 7.83(1H,s,$D_2O$-Disappearance), 8.24(1H,s), 8.38(1H,s)

Example 4

14.3 g of 6-chloro-9-β-D-ribofuranosyl-9H-purine and 15 g of triphenylchlorosilane were dissolved in 500 ml of pyridine and stirred for one hour at room temperature. Pyridine was distilled off and the residue was dissolved in benzene. The benzene layer was washed with 1N HCl and brine, and then dried over anhydrous sodium sulfate. The solvent was distilled off and the residue was recrystallized from a mixture of hexane and ethyl acetate to give 21.5 g of 6-cholor-9-(5-O-triphenylsilyl-β-D-ribofuranosyl)-9H-purine.

5.45 g of the resulting product was dissolved in 50 ml of ethyl iodide and silver oxide was added thereto with heating. The reaction mixture was applied on a silica gel column. The column was washed with

benzene and eluted with a mixture of hexane and ethyl acetate. The eluate was concentrated to dryness under reduced pressure. The residue was dissolved in benzene and a 40%(w/v) aqueous solution of monomethylamine was added thereto. After stirring overnight, the benzene layer was collected, washed with 1N HCl and brine and purified by silica gel column chromatography to give 6-amino-9-(5-O-triphenylsilyl-β-D-ribofuranosyl)-9H-purine. The resulting product was dissolved in tetrahydrofuran and tetra-n-butylammonium fluoride in tetrahydrofuran was added thereto. After stirring for 30 minutes at room temperature, the solution was concentrated to dryness under reduced pressure and purified by silica gel column chromatography to give 600 mg of $N^6$-methyl- 2',3'-O-diethyladenosine (Compound 61). m.p.: amorphous
1H-NMR (MeOH-$d_4$):
1.07(3H,t), 1.22(3H,t), 3.08(3H,s), 3.48(1H,m), 3.58(1H,m), 3.65(2H,m), 3.70(1H,dd), 3.87(2H,m), 4.20(1H,dd), 6.00(1H,d), 8,20(1H,s), 8.23(1H,s)

Example 5

2-Methyladenosine was dialkylated in the same manner as in Example 2 to give 2,2',3'-O-trimethyladenosine (Compound 63). m.p.: 156 ° C
1H-NMR (DMSO-$d_6$): 2.39(3H,s), 3.28(3H,s), 3,40(3H,s), 3.58 (1H,m), 3.70(1H,m), 4.08(1H,m), 4.13(1H,m), 4.52(1H,m), 5.80(1H,m,$D_2O$-Disappearance), 5.95(1H,d), 7.27(2H,s,$D_2O$-Disappearance), 8.28(1H,s)

Example 6

Compound 63 was methylated by methyl iodide and then rearranged to give 2,$N^6$,2',3'-O-tetramethyladenosine (Compound 64).
1H-NMR (DMSO-$d_6$):
2.43(3H,s), 2.94(3H,s), 3.28(3H,s), 3.40(3H,s), 3.57(1H,m), 3.70(1H,m), 4.08(1H,m), 4.13(1H,m), 4.52(1H,m), 5.79(1H,m,$D_2O$-Disappearance), 5.96(1H,d), 7.71(1H,s,$D_2O$-Disappearance), 8.26(1H,s)

Example 7

1.2 g of 2-iodoadenosine was suspended in 150 ml of 1 mmol tin chloride dihydrate/methanol. 50 ml of 0.4-0.5M diazomethane in 1,2-dimethoxyethane was added under stirring. After stirring for one hour at room temperature, the reaction mixture was concentrated to dryness under reduced pressure. The resulting product was applied on an ODS column and eluted with 40%(v/v) methanol in 0.1%(v/v) aqueous solution of TFA. First, 2-iodo-2'-O-methyladenosine (Compound 47) was eluted, and then 2-iodo-3'-O-methyladenosine (Compound 48) was eluted. Both fractions were concentrated to dryness to give 135 mg of compound 47 and 56 mg of Compound 48.
2-iodo-2'-O-methyladenosine (Compound 47)
1H-NMR (D2O): 3.52(3H,s), 3.92(1H,dd), 3.97(1H,dd), 4.27(1H,m), 4.54(1H,dd), 4.61(1H,dd), 6.02(1H,d), 7.98(1H,s)
2-iodo-3'-O-methyladenosine (Compound 48)
1H-NMR (D2O): 3.58(3H,s), 3.86(1H,dd), 3.94 (1H,dd), 4.18(1H,dd), 4.35(1H,m), 4.97(1H,dd), 5.93(1H,d), 7.94(1H,s)
In the same manner by using 2-substituted adenosines, $N^6$-substituted adenosines or 2,$N^6$-disubstituted adenosines, the following compounds were obtained.
2-Methoxy-3'-O-methyladenosine (Compound 9)
1H-NMR ($D_2O$): 3.56(3H,s), 3.84(1H,dd), 3.94(1H,dd), 3.96(3H,s), 4.22(1H,dd), 4.33(1H,m), 5.06(1H,dd), 5.98-(1H,d), 8.10(1H,s,8-H)
$N^6$,2'-O-Dimethyl-2-hexyladenosine (Compound 30)
1H-NMR (MeOH-$d_4$): 0.88(3H,t), 1.33(6H,m), 1.78(2H,quintet), 2.74(2H,t), 3.12(3H,brs), 3.37(3H,s), 3.73-(1H,dd), 3.88(1H,dd), 4.18(1H,m), 4.49(2H,m), 5.98(1H,d), 8.10(1H,s,8-H)
$N^6$,2'-O-Dimethyl-2-decyladenosine (Compound 31)
1H-NMR (MeOH-$d_4$): 0.88(3H,t), 1.27-1.33(14H,m), 1.78(2H,quintet), 2.75(2H,t), 3.13(3H,brs), 3.37(3H,s), 3.73(1H,dd), 3.89(1H,dd), 4.18(1H,m), 4.49(2H,m)
$N^6$,2'-O-Dimethyl-2-(1-hexyn-1-yl)adenosine (Compound 32)
1H-NMR (MeOH-$d_4$): 0.97(3H,t), 1.52(2H,m), 1.62(2H,m), 2.45(2H,t), 3.09(3H,brs), 3.40(3H,s), 3.74(1H,dd), 3.88(1H,dd), 4.15(1H,m), 4.42(1H,dd), 4.48(1H,dd), 6.00(1H,d), 8.22(1H,s)
$N^6$,2'-O-Dimethyl-2-(1-dodecyn-1-yl)adenosine (Compound 33)
1H-NMR (MeOH-$d_4$): 0.89(3H,t), 1.30(14H,m), 1.49(2H,m), 1.63(2H,quintet), 2.43(2H,t), 3.41(3H,s), 3.74-

(1H,dd), 3.88(1H,dd), 4.15(1H,m), 4.41(1H,dd), 4.48(1H,dd), 6.00(1H,d), 8.30(1H,s)
2-Phenyl-2'-O-methyladenosine (Compound 37)
1H-NMR (MeOH-$d_4$): 3.53(3H,s), 3.77(1H,dd), 3.91(1H,dd), 4.12(1H,m), 4.50(1H,dd), 4,54(1H,dd), 6.20(1H,d), 7.43(3H,m), 8.32(2H,m), 8.36(1H,s)
2-Phenyl-3'-O-methyladenosine (Compound 38)
1H-NMR (MeOH-$d_4$): 3.53(3H,s), 3.74(1H,dd), 3.88(1H,dd), 4.10(1H,dd), 4.22(1H,m), 5.00(1H,dd), 6.07(1H,d), 7.42(3H,m), 8.32(3H,m)
2-Bromo-2'-O-methyladenosine (Compound 43)
1H-NMR ($D_2O$): 3.50(3H,s), 3.91(1H,dd), 3.99(1H,dd), 4.37(1H,m), 4.57(1H,dd), 4.67(1H,dd), 6.13(1H,d), 8.37-(1H,s)
2-Bromo-3'-O-methyladeonsine (Compound 44)
1H-NMR ($D_2O$): 3.60(3H,s), 3.90(1H,dd), 4.01(1H,dd), 4.19(1H,dd), 4.44(1H,m), 4.95(1H,dd), 6.07(1H,d), 8.37-(1H,s)
2-Bromo-$N^6$,2'-O-dimethyladenosine (Compound 45)
1H-NMR (D2O): 3.22(3H,s), 3.57(3H,s), 3.90(1H,dd), 3.98(1H,dd), 4.29(1H,m), 4.59(1H,dd), 4.65(1H,dd), 6.12-(1H,d), 8.16(1H,s)
2-Bromo-$N^6$,3'-O-dimethyladenosine (Compound 46)
1H-NMR ($D_2O$): 3.07(3H,s), 3.60(3H,s), 3.93(1H,dd), 3.99(1H,dd), 4.23(1H,dd), 4.36(1H,m), 5.03(1H,dd), 6.03-(1H,d), 8.14(1H,s)
2-Pentylamino-2'-O-methyladenosine (Compound 52)
1H-NMR (MeOH-$d_4$): 0.94(3H,t), 1.38(4H,m), 1.65(2H,m), 3.42(2H,t), 3.49(3H,s), 3.76(1H,dd), 3.87(1H,dd), 4.08(1H,m), 4.25(1H,t), 4.43(1H,t), 5.99(1H,d), 8.22(1H,s)
2-Phenylamino-2'-O-methyladenosine (Compound 53)
1H-NMR (MeOH-$d_4$): 3.46(3H,s), 3.74(1H,dd), 3.85(1H,dd), 4.07(1H,m), 4.23(1H,dd), 4.39(1H,dd), 6.03(1H,d), 7.11(1H,t), 7.35(2H,t), 7.60(2H,d), 8.35(1H,s)
2-Phenylamino-3'-O-methyladenosine (Compound 54)
1H-NMR (MeOH-$d_4$): 3.46(3H,s), 3.69(1H,dd), 3.79(1H,dd), 3.94(1H,dd), 4.16(1H,m), 4.74(1H,dd), 5.91(1H,d), 7.13(1H,t), 7.36(2H,t), 7.60(2H,d), 8.28(1H,s)
2-Phenylamino-$N^6$,2'-O-dimethyladenosine (Compound 55)
1H-NMR (MeOH-$d_4$): 3.16(3H,s), 3.47(3H,s), 3.77(1H,dd), 3.88(1H,dd), 4.11(1H,m), 4.20(1H,dd), 4.40(1H,dd), 6.06(1H,d), 7.12(1H,t), 7.35(2H,t), 7.63(2H,d), 8.42(1H,s)
2-Phenylamino-$N^6$,3'-O-dimethyladenosine (Compound 56)
1H-NMR (MeOH-$d_4$): 3.10(3H,s), 3.49(3H,s), 3.72(1H,dd), 3.85(1H,dd), 3.98(1H,dd), 4.17(1H,m), 4.85(1H,dd), 5.88(1H,d), 6.94(1H,t), 7.26(2H,t), 7.69(2H,d), 7.99(1H,s)
2-(3-Hydroxy-1-propyn-1-yl)-2'-O-methyladenosine (Compound 57)
1H-NMR ($D_2O$): 3.45(3H,s), 3.86(1H,dd), 3.95(1H,dd), 4.32(1H,m), 4.51(1H,dd), 4.52(2H,s), 4.62(1H,dd), 6.08-(1H,d), 8.30(1H,s)
2-(3-Hydroxy-1-propyn-1-yl)-3'-O-methyladenosine (Compound 58)
1H-NMR ($D_2O$ + MeOH-$d_4$): 3.51(3H,s), 3.80(1H,dd), 3.92(1H,dd), 4.09(1H,m), 4.36(1H,m), 4.47(2H,s), 4.83-(1H,dd), 5.96(1H,d), 8.30(1H,s)

Example 8

2.2 g of AICA-2'-O-methylriboside was dissolved in 20 ml of dimethylformamide and 1.7 g of benzoylisothiocyanate was added thereto. After stirring for 3 hours at room temperature, 3.3 g of dicyclohexylcarbodiimide was added and reacted for 20 hours at room temperature. The reaction mixture was concentrated to an oily residue. To the residue, 50 ml of ethanol and 50 ml of aqua ammonia were added, and stirred for 18 hours at room temperature. The resulting precipitate was collected by filtration to give 1.7 g of 2-hydroxy-2'-O-methyladenosine (Compound 39). 1H-NMR (D2O): 3.46(3H,s), 3.82(1H,dd), 3.90(1H,dd), 4.27(1H,m), 4.50(1H,dd), 4.59(1H,dd), 5.95(1H,d), 8.03(1H,s)
In the same manner, AICA-3'-O-methylriboside was used as a starting material to give 2-hydroxy-3'-O-methyladenosine (Compound 40).
1H-NMR (D2O): 3.54(3H,s), 3.82(1H,dd), 3.94(1H,dd), 4.10(1H,dd), 4.36(1H,m), 4.86(1H,dd), 5.89(1H,dd), 8.03(1H,s)
The following description serves to illustrate the pharmaceutical activity of the compounds of the present invention.

1. Adenosinedeaminase Inhibiting Action.

The enzymatic reaction was conducted at 25 °C in a 0.05M phosphate buffer (pH = 7.5). Thus, a reaction solution comprising 800 µl of substrate solution (adenosine), 100 µl of a solution to be tested and 100 µl of an enzyme solution (adenosinedeaminase of type VII mucous membrane of the calf intestinal tract) was made to react for 5 minutes and the reaction was stopped by adding 100 µl of acetic acid. Then the amount of inosine produced was determined by means of HPLC to measure the inhibiting activity. The test was conducted for various substrate concentrations and Ki values were measured by Lineweaver-Burk plots.

Examples of the results are given in Table 1.

TABLE 1

| Compound tested | Ki Value (M) | Compound tested | Ki Value (M) |
|---|---|---|---|
| 1 | $1.31 \times 10^{-5}$ | 39 | $1.90 \times 10^{-5}$ |
| 2 | $4.93 \times 10^{-4}$ | 43 | $3.20 \times 10^{-6}$ |
| 4 | $3.47 \times 10^{-6}$ | 44 | $2.20 \times 10^{-5}$ |
| 5 | $2.46 \times 10^{-5}$ | 45 | $1.20 \times 10^{-7}$ |
| 6 | $1.79 \times 10^{-4}$ | 46 | $3.70 \times 10^{-6}$ |
| 18 | $1.24 \times 10^{-6}$ | 47 | $2.30 \times 10^{-7}$ |
| 19 | $7.78 \times 10^{-5}$ | 48 | $4.40 \times 10^{-6}$ |
| 20 | $2.69 \times 10^{-4}$ | 50 | $2.80 \times 10^{-5}$ |
| 21 | $3.30 \times 10^{-7}$ | 51 | $5.00 \times 10^{-6}$ |
| 22 | $5.58 \times 10^{-8}$ | 52 | $6.00 \times 10^{-7}$ |
| 23 | $8.90 \times 10^{-7}$ | 53 | $1.70 \times 10^{-7}$ |
| 24 | $1.10 \times 10^{-7}$ | 54 | $2.20 \times 10^{-6}$ |
| 25 | $3.59 \times 10^{-4}$ | 55 | $1.30 \times 10^{-8}$ |
| 27 | $8.59 \times 10^{-7}$ | 56 | $1.10 \times 10^{-7}$ |
| 30 | $9.00 \times 10^{-8}$ | 57 | $3.00 \times 10^{-7}$ |
| 31 | $2.80 \times 10^{-7}$ | 59 | $7.50 \times 10^{-6}$ |
| 32 | $1.20 \times 10^{-7}$ | 60 | $7.50 \times 10^{-7}$ |
| 33 | $1.50 \times 10^{-5}$ | 61 | $1.50 \times 10^{-7}$ |
| 34 | $3.59 \times 10^{-4}$ | 63 | $1.30 \times 10^{-6}$ |
| 37 | $6.60 \times 10^{-7}$ | 64 | $8.80 \times 10^{-7}$ |
| 38 | $7.90 \times 10^{-6}$ | 73 | $1.30 \times 10^{-3}$ |

2. Therapeutic Action to Nephritis.

When puromycin aminonucleoside is administered to rats, symptoms similar to protein-rich urine, hypoproteinemia, hyperlipemia, nephrotic syndrome, etc. result and, therefore, rats which were administered with puromycin aminonucleoside have been used as pathological model animals for nephritis. A method by Endo, et al. [Sogo Rinsho, vol.38, no.5, page 821 (1989)] was somewhat modified and used as a test method here. Thus, a solution of puromycin aminonucleoside was dissolved in a physiological saline solution and administered just once into the tail vein of male rats (SD strain) of about 200 g body weight at a dose of 100 mg/kg (the initial or zero-th day).

The compound to be tested was dissolved in a physiological saline solution and was given orally for consecutive five days from the zero-th day at a dose of 50 mg/kg each. After 24 hours, the accumulated urine was collected and the amount of urine and that of protein in the urine were measured. Blood was collected on the tenth day and total protein in serum, creatinine in serum and urea nitrogen were measured.

Examples of the results are given in Tables 2 and 3.

TABLE 2

| Compound to be tested | Urea nitrogen (mg/dl) | Total protein in serum (g/dl) | Serum creatinine (mg/dl) |
|---|---|---|---|
| (Normal) | 17.6 | 7.90 | 0.38 |
| (Control) | 40.0 | 7.08 | 0.85 |
| Compound 27 | 31.9 | 7.44 | 0.71 |

TABLE 3

| Days | Amount of protein in urine (mg/day) | |
|---|---|---|
| | Control | Compound 27 |
| 1st day | 0 | 0 |
| 2nd | 32.0 | 29.2 |
| 3rd | 44.7 | 33.8 |
| 4th | 173.4 | 122.5 |
| 5th | 522.5 | 399.8 |
| 6th | 704.1 | 443.7 |
| 7th | 826.5 | 558.7 |
| 8th | 811.6 | 595.6 |
| 9th | 814.0 | 593.5 |
| 10th | 684.0 | 528.6 |

3. Inhibitory Action against Generation of Activated Oxygen

Human peripheral polymorphonuclear leukocytes (PML, 2 x $10^6$ cells) prepared in a conventional method, bovine heart cytochrome C type-III (75 nmol), cytocharasin (5 $\mu$g) and the tested drug were mixed with HEPES-buffered saline solution (final amount 1 ml) and incubated for 5 minutes at 37 °C. N-Formyl-methionyl-leucyl-phenylalanine (FMLP) was added (final concentration $10^{-7}$ M) and incubated for 5 minutes. Immediately after incubation, the reaction mixture was centrifuged at 4 °C, and then the absorbance of the supernatant at 550 nm was measured with a spectrophotometer.

An excess amount of bovine liver superoxide dismutase (SOD) was added to said reaction mixture and the absorbance of the supernatant was also measured in the same manner as above as a blank value.

A portion of adenosinedeaminase (ADA) may be removed during the preparation of human peripheral PML. Therefore, the inhibitory action of the tested drug against the generation of superoxide was measured in the same manner as mentioned above, adding 0.02 units of bovine liver ADA with human peripheral PML.

The inhibition of the superoxide generation was calculated by the following equation, and examples of the results are given in Table 4.

$$\text{Inhibition}\ (\%) = \left(1 - \frac{\text{Drug}\ A_{550} - \text{SOD}\ A_{550}}{\text{Control}\ A_{550} - \text{SOD}\ A_{550}}\right) \times 100$$

TABLE 4

| Without ADA | | With ADA | |
|---|---|---|---|
| Compound 27 (M) | Inhibition | Compound 27 (M) | Inhibition |
| $1 \times 10^{-3}$ | 100% | $1 \times 10^{-4}$ | 88% |
| $1 \times 10^{-4}$ | 79% | $1 \times 10^{-5}$ | 77% |
| $1 \times 10^{-5}$ | 22% | $1 \times 10^{-6}$ | 34% |
| $1 \times 10^{-6}$ | 2% | $1 \times 10^{-7}$ | 10% |

4. Suppressive Action against Ischemic Edema

The right hind paw of ICR-strain male mice (11 weeks old) was fastened by a rubber band to stop blood circulation in the paw for 20 minutes, and then the rubber band was removed to recover blood circulation. The tested drug was administered intravenously before said treatment. Each of the right and left hind paws was weighed and the suppressive action was measured according to the weight difference between treated and untreated paws. Examples of the results are given in Table 5.

TABLE 5

| Tested Drug (10 mg/kg) | Paw Weight (mg) | | Inhibition (%) |
|---|---|---|---|
| | Treated | Untreated | |
| Control | 314.8 ± 6.8 | 219.9 ± 6.2 | - |
| Compound 27 | 254.4 ± 6.6 | 223.8 ± 6.2 | 67.7 |
| Allopurinol | 283.2 ± 6.3 | 222.9 ± 6.9 | 36.4 |

5. Effect on the Concentrations of Adenosine and Inosine

Human peripheral polymorphonuclear leukocytes ($2 \times 10^6$ cells), cytocharasin (5 $\mu$g) and the tested drug were mixed with HEPES-buffered saline solution (final amount 0.5 ml) and incubated for 5 minutes. N-Formyl-methionyl-leucyl-phenylalanine (FMLP) was added (final concentration $10^{-7}$ M) and incubated for 10 minutes. As a result of HPLC analyses of the reaction mixture, in the FMLP-treated group, the inosine peak increased compared with the control group. The compound 27 of the precent invention was added to the FMLP-treated group, which resulted in a significant decrease of the inosine peak and increase of the adenosine peak as compared with a group wherein the compound of the present invention was not added.

As shown in the above results (cf. Table 1), the compounds of the present invention exhibit an excellent adenosinedeaminase inhibiting action. Adenosinedeaminase which is a metabolic enzyme for adenosine is inhibited whereby the adenosine concentration in tissues increases. Neutrophils produce activated oxygen when the tissue is in an ischemic state and adenosine inhibits said activated oxygen production and, in addition, it directly eliminates the produced activated oxygen. Further, it lowers the inosine concentration whereby it decreases the supply of hypoxanthine which is a substrate of the xanthine-xanthineoxidase system (which is one of the systems producing the activated oxygen).

Adenosinedeaminase inhibiting substances having an oxygen production inhibiting action and an eliminating action for an activated oxygen source as such show pharmacological actions such as the improvement of coronary and cerebral blood vessel circulation, prevention and therapy of renal diseases, anti-inflammatory activity, etc.

Further, as shown in Tables 2 and 3, the compounds of the present invention having adenosinedeaminase inhibiting action were evaluated by means of pharmacological experiments using pathological model animals for nephritis induced by puromycin aminonucleoside, taking protein in urine, total protein in serum, creatinine in serum, urea nitrogen, etc. as indexes whereby the therapeutic effect was clearly noted.

Consequently, the compounds of the present invention having adenosinedeaminase inhibiting action are useful as pharmaceuticals for prevention and therapy of various kinds of diseases such as ischemic heart

diseases (e.g. angina pectoris, myocardial infarction and arrhythmia), diseases caused by cerebrovascular disorders (e.g. cerebral hemorrhage, cerebral infarction, cerebral apoplexy and after-effects thereof and cerebral arteriosclerosis), renal diseases (e.g. nephritis and renal failure) and allergic diseases (e.g. asthma, allergic rhinitis, allergic conjunctivitis, urticaritis and rheumatism). Moreover, they are very useful as pharmaceuticals for prevention and therapy of post-operative complicated diseases because they inactivate activated oxygen which is generated in ischemic areas during the recirculation of blood after operations.

Adenosine analogs such as 3'-deoxyadenosine and xylosyladenine (anticancer drugs) and arabinosyladenine (exhibiting antiherpes activity) are easily deaminated by adenosinedeaminase in vivo and thus become inactive. Accordingly, when the compounds of the present invention having adenosinedeaminase inhibiting action are administered before or together with administration of the above-mentioned anticancer drugs or antiviral drugs, an effect of inhibiting the decrease in action of such adenosine analogous anticancer and antiviral drugs can be expected as well.

Adenosine itself has many pharmacological activities such as a cardiovasodilating or platelet-aggregation inhibiting activity. Thus, it is used as a drug improving blood circulation to treat heart failure, myocardial infarction, etc. Adenosine is metabolized by adenosinedeaminase, and is consequently inactivated. Accordingly, in the same way as above, when the compounds of the present invention are administered before or together with the administration of adenosine, the instant compounds may inhibit the decrease of said activities of adenosine. It is therefore advantageous to administer adenosine in combination with a compound of the present invention.

The compounds of the present invention can be made into pharmaceuticals by combining with a suitable pharmaceutically acceptable carrier or diluent for medical use and can be made into pharmaceutical preparations by any of conventional methods giving solid, semisolid, liquid or gaseous forms for oral or parenteral administration. In manufacturing such preparations, the compounds of the present invention may be used in a form of pharmaceutically acceptable salts thereof or the compound of the present invention may be used either solely or jointly in a form of a suitable combination. Alternatively, the compound may be compounded with other pharmaceutically active components.

In the case of preparations for oral use, the compound of the present invention may be made into tablets, diluted powders, granules or capsules with or without suitable additives such as conventional fillers (e.g. lactose, mannitol, corn starch and potato starch) as well as binders (e.g. crystalline cellulose, cellulose derivatives, gum arabic, corn starch and gelatin), lubricants (e.g. talc and magnesium stearate), extenders, moisturizers, disintegrating agents, preservatives, perfumes, etc. Alternatively, the compound of the present invention may be mixed with base material of fat/oil type (e.g. cacao butter), an emulsifying base material, water-soluble base material (e.g. Macrogel), a hydrophilic base material, etc. to give an ointment.

In the case of injections, the compound may be made into a solution or a suspension in aqueous solvents or nonaqueous ones such as distilled water for injection, physiological saline liquid, Ringer solution, plant oil, synthetic fatty acid glycerides, higher fatty acid esters, propylene glycol, etc. Further, depending upon the state of the patient, or the type of the disease, the compound may be made into other preparation forms which are most suitable for the therapy such as inhalants, aerosols, ointments, poultices, eye drops, etc. The desired dosage of the compound of the present invention may vary depending upon the patient to be treated, the preparation form, method of administration, period for administration, etc., though, in general, 0.1 to 5,000 mg or, preferably, 0.2 to 3,000 mg per day may be given to an adult by oral route for achieving the desired effect.

In the case of parenteral administrations such as injections, there are many cases where a lower dose than the above by oral administration to an extent of from 1/3 to 1/10 is desired because of the influence of absorption, etc.

## Claims

1. The use of a compound represented by the following general formula (I) or a pharmaceutically acceptable salt thereof

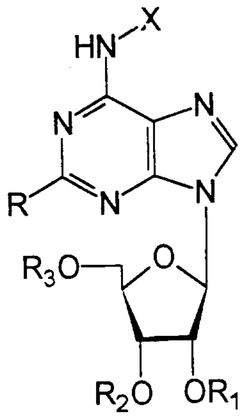

(I)

wherein each of $R_1$, $R_2$ and $R_3$ which may be same or different is hydrogen or alkyl;
R is hydrogen, alkyl, alkenyl, alkynyl, hydroxyalkynyl, alkoxy, phenyl, hydroxy, amino, alkylamino, phenylamino or halogen;
X is hydrogen, alkyl, alkynyl, allyl, methallyl, cycloalkyl, an alkyl group being substituted with one or more hydroxy groups, phenyl (which may be substituted with one or more halogen atoms, lower alkyl groups, lower alkoxy groups and/or trifluoromethyl groups), an alkyl group being substituted with one or more phenyl groups (which may be substituted with one or more halogen atoms, lower alkyl groups, lower alkoxy groups and/or trifluoromethyl groups), bicycloalkyl, naphthylalkyl, acenaphthylenylalkyl or a group represented by the following general formulae (II) or (III)

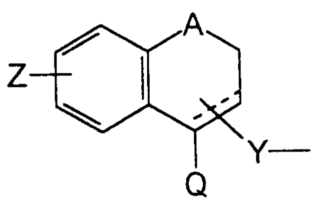

(II)

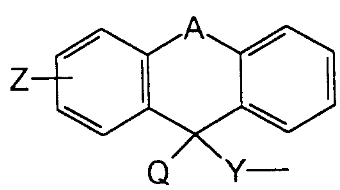

(III)

wherein Z is hydrogen, hydroxy or lower alkoxy;
Q is hydrogen or hydroxy;
A is $-CH_2-$, -O-, -S- or a single bond;
Y is $-(CH_2)_n-$ or a single bond with n being an integer of 1 to 3;
a dashed line means a single bond or a double bond;
and wherein at least one of $R_1$, $R_2$ and $R_3$ is alkyl,
for the preparation of a medicament which is an adenosinedeaminase inhibitor.

**2.** The use according to claim 1 wherein $R_3$ is hydrogen.

**3.** The use according to claim 2 wherein X is hydrogen.

**4.** The use according to claim 2 wherein X is an alkyl group having 1 to 3 carbon atoms.

**5.** Adenosine derivatives represented by the following general formula (Ia) or pharmaceutically acceptable salts thereof

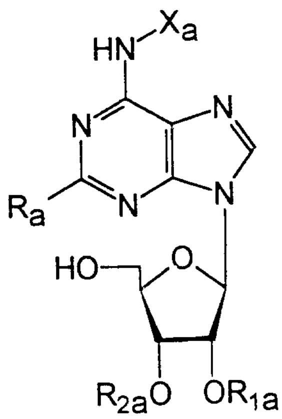

(Ia)

wherein each of $R_{1a}$, $R_{2a}$ and $X_a$ may be same or different and is hydrogen or alkyl; $R_a$ is alkyl having more than 6 carbon atoms, alkenyl, alkynyl, hydroxyalkynyl, alkoxy, phenyl, hydroxy, alkylamino or phenylamino; and at least one of $R_{1a}$ and $R_{2a}$ is alkyl.

**6.** Adenosine derivatives represented by the following general formula (Ib) or pharmaceutically acceptable salts thereof,

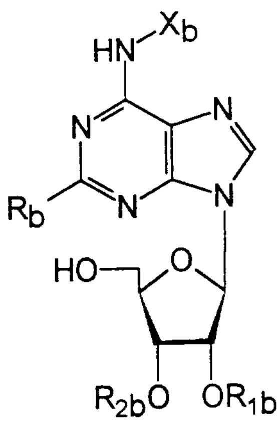

(Ib)

wherein each of R1b and R2b may be same or different and is alkyl; Rb and Xb is hydrogen or alkyl; and at least one of Rb and Xb is alkyl.

**7.** Adenosine derivatives represented by the following general formula (Ic) or pharmaceutically acceptable salts thereof,

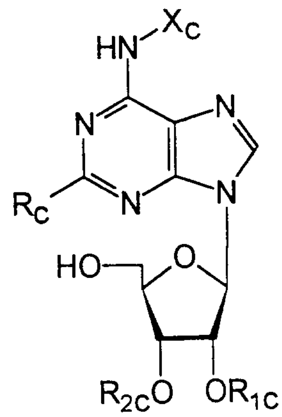

(Ic)

wherein each of $R_{1c}$, $R_{2c}$ and $X_c$ may be same or different and is hydrogen or alkyl; $R_c$ is bromo or iodo; and at least one of $R_{1c}$ and $R_{2c}$ is alkyl.

**8.** Adenosine derivatives represented by the following general formula (Id) or pharmaceutically acceptable salts thereof

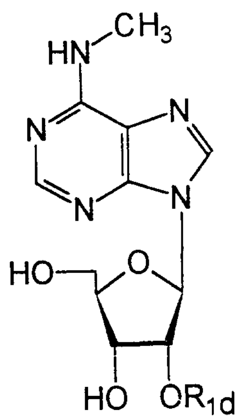

(Id)

wherein $R_{1d}$ is an alkyl group having more than 2 carbon atoms.

**9.** Pharmaceutical composition comprising at least one compound of formulae (Ia), (Ib), (Ic) or (Id) or a pharmaceutically acceptable salt thereof according to anyone of claims 5 to 8 in combination with a pharmaceutically acceptable carrier or diluent.

**10.** The use of a compound of formulae (Ia), (Ib), (Ic) or (Id) or a pharmaceutically acceptable salt thereof according to anyone of claims 5 to 8 for the preparation of a medicament which is an adenosinedeaminase inhibitor.

**11.** The use according to anyone of claims 1-4 and 10 wherein the medicament is for the prevention or therapy of ischemic heart diseases, diseases caused by cerebrovascular disorders, renal diseases and allergic diseases and of post-operative complicated diseases involved with activated oxygen which is generated in ischemic areas during the recirculation of blood after operations.

**12.** A medicament, comprising a combination of

(A) a compound of formula (I) as defined in claim 1 which acts as an adenosinedeaminase inhibitor and

(B) adenosine or a therapeutically useful adenosine analogous drug which is different from the compounds of formula (I),

as active ingredients.

**13.** The medicament of claim 12, wherein the adenosine analogous drug is an anticancer and/or an antiviral drug.

**14.** The medicament of claim 13, wherein the anticancer drug is 3'-deoxyadenosine or xylosyladenine and the antiviral drug is arabinosyladenine.